# EUROPEAN PATENT SPECIFICATION

(11) **EP 3 966 549 B1**
(45) Date of publication and mention of the grant of the patent: **21.08.2024**
(21) Application number: 20735238.6
(22) Date of filing: 08.05.2020
(51) Int. Cl.: G01N 11/12

(54) **DEVICE FOR ANALYSIS OF WAXES**
VORRICHTUNG ZUR ANALYSE VON WACHSEN
DISPOSITIF POUR L'ANALYSE DE CIRES

(30) Priority: 09.05.2019 PL 42988119
(43) Date of publication of application: 16.03.2022
(73) Proprietor: Uniwersytet Marii Curie Sklodowskiej, 20-031 Lublin (PL)
(72) Inventor: GAGOS , Mariusz, 20-530 Lublin (PL); PIETROW, Marek, 20-330 Lublin (PL); WAWRYSZCZUK, Jan, 20-032 Lublin (PL)
(74) Representative: Augustyniak, Magdalena Anna
(86) International application number: PCT/IB2020/054372
(87) International publication number: WO 2020/225786

(56) References cited:
- EP-A1- 0 894 258
- SU-A1- 1 784 864
- US-A- 2 535 830
- K. K. CHEE ET AL: "A study of low shear viscosity of polymer melts", CANADIAN JOURNAL OF CHEMICAL ENGINEERING, vol. 48, no. 4, 1 August 1970 (1970-08-01), US, pages 362 - 372, XP055718894, ISSN: 0008-4034, DOI: 10.1002/cjce.5450480404

## Description

The invention relates to a device for analysis of beeswax, cosmetic waxes or waxes used in pharmaceutical formulations and articles like all kinds of candles or grave lanterns, designed to discern between the natural wax and waxes modified by substances which reduce its quality, which is easy to operate, portable, able to be commonly used in case of need.

In consideration of growing prices of the natural beeswax, one of the numerous problems of the modern bee-keeping is modifying such a wax with additives. A modified wax, used by beekeepers for a low-quality foundation, produces an effect of deteriorating conditions of bee families, as well as lowering the quality of other products made of it.

Among known and widely applied methods of assessing wax quality an organoleptic method is used, which, unfortunately, identifies its adulterations unsatisfactorily. Other methods which enable unequivocal detection of adulteration of wax, is e.g. gas chromatography coupled with mass spectrometry (GCMS), FTIR spectroscopy, scanning calorimetry (DSC) or X-ray diffraction, however, such analyses are carried out in laboratories on a specialized equipment, restricting their availability to foundation makers and beekeepers.

There are a number of technical solutions that allow to test samples properties based on the penetration of the blade into the sample under the influence of a controlled pressure force, the temperature of which can be simultaneously controlled. The features of the devices are directed to the properties of the materials tested mainly resins and thermoplastic polymers.

Document US2535830 A discloses the apparatus intended for testing the thermo-setting materials such as resins that comprises a base for supporting a sample an arm spaced above the base, and indicator on the arm, a vertically slidable plunger carried by the connecting the plunger with the indicator, a needle depending from the plunger for pressure contact with the sample, and a heater for holding the needle at a substantially even degree of heat. The device measures the extent to which the needle will penetrate a sample, whereas the electric heater heat not only the needle but also the weigh element to which the needle is attached.

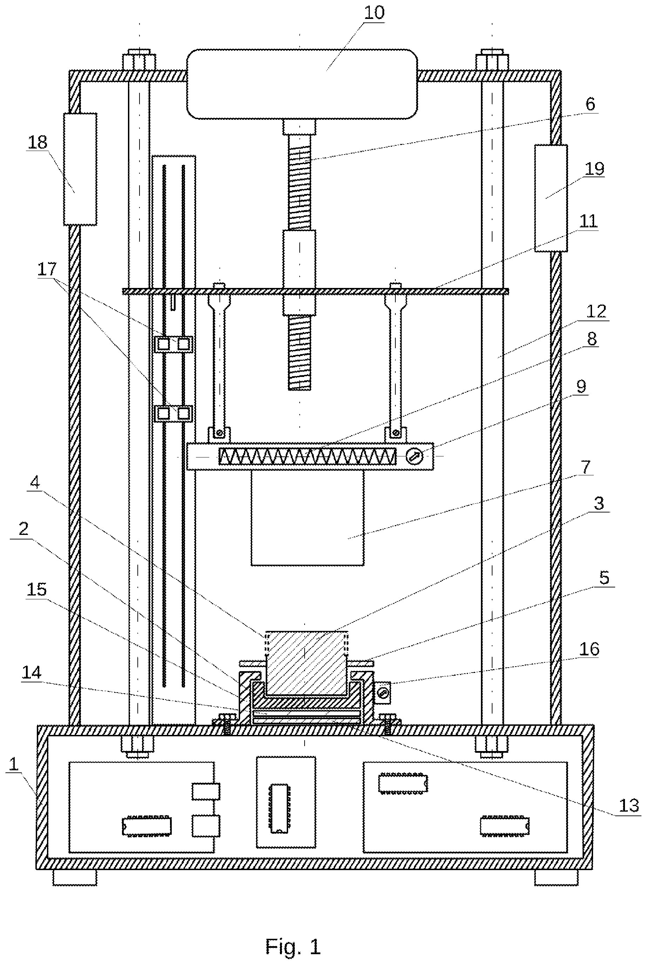

Document SU 1 784 864 A1 (figure 1) discloses the apparatus intended for testing thermoplastic materials, having a base with the fastening device for a model sample, the loader of the calibrated power load on the road, the rod heated by the electric current, the speed matter of the displacement of a rod, electric current source, a gouge of the electric current passing through the rod, and variable resistor for regulating the force of the electric current passing through the rod. The device assumes the movement of a rod penetrating the sample with uniform speed, which entails the need to heat it to a temperature above the melting point of the tested material. The pressure on the sample is achieved by applying the restoring force.

Document K.K. CHEE ET Al: "A study of low shear viscosity of polymer melts", CANADIAN JOURNAL OF CHEMICAL ENGINEERING vol. 48, no. 4, 1 August 1970 (1970-08-01), pages 362-372 (figure 1) discloses coaxial cylinder viscometer for testing polymers in a fluid state where the temperature of the sample is stabilized to the temperature of the device and the needle.

Document EP0894258 A1 discloses rheometer for measuring viscosity at near-zero share rate of high solids suspensions and is provided with the moving road with a ball shaped tip fastened at the end thereof, that is traveling vertically downwards at a preselected constant speed via linearly movable slide member mounted on a slide guide towards the tested sample placed in the container. The device measure the maximum force exerted on said ball tip as the rode move through the portion of the sample at a predetermined constant speed.

The object of the invention was to provide a portable device, easy to operate and able to be commonly used in case of need, serving to discern between the natural wax and a wax doped with substances which reduce its quality, by measuring physical properties discriminating them.

The device for analysis of waxes according to the invention is constructed of a base with a display fixed on its front panel and an internal chamber, where a power unit with a microcontroller to control operation of the device is provided, to record a measurement result and send it to the display. At the upper surface of the base a holder is provided with a support for a container with a tested wax sample having vertical incisions located symmetrically on opposite faces of the side surface to facilitate passage of an element penetrating the analyzed wax sample, under which incisions shields are arranged to protect the lower part of the holder against contamination with the wax, and above it vertically in the middle a mechanism is installed in a form of a tractive screw connected with a stepper motor, and terminated at the bottom with the sample-penetrating element with a heater with a temperature sensor arranged above it. Precise sliding movement of the element is controlled by the motor and stabilized by two arms of a guide moving along vertical rods attached to the base. At the bottom of the holder, on a gasket, a force sensing device is installed, which is connected to the microcontroller and records a measurement time-variable value of the pressure force of the penetrating element on the tested wax sample, the variability characteristics thereof permitting to conclude about presence of the additives in the natural wax. All pieces of the holder are shielded with a panel fixed to the base and provided with a temperature sensor also connected to the microcontroller. A post is also mounted on the base with two optocouplers for determining the time of penetration of the element through the sample. The whole is enclosed in a casing with an opening window at the front panel, to provide access to the holder designed for inserting the sample.

It is preferred that the element penetrating the wax sample is in a shape of a horizontally fixed rod, tube, plane such as e.g. a knife or a vertical needle or a rod with a sharpened tip.

It is also preferred that the device casing is provided with open-work walls with a fan and a Peltier module with a heater, controlled by the microcontroller-for faster cooling-down the interior of the analyzer to a programmed temperature to start a subsequent measurement.

An electronic circuit in a form of the properly programmed microcontroller controls also the pressure force of the element penetrating the sample, and also the temperature of a coil heating the element penetrating the sample indicated by the sensor, within the wax softening temperature range, and stabilizes the temperature of the entire system after completion of the analysis indicated by the sensor provided in the holder area, and makes it return to the starting value before a subsequent measurement. In addition, the system records and analyzes the penetration time of the predetermined thickness of the sample and the pressure force of the penetrating element and the sample container on the force sensing device, which varies through the measurement period, depending on the kind and content of analyzed sample components.

Pure natural waxes are characterized by approximate physical features such as an elasticity or viscosity at the given temperature conditions, a melting point, and also a thermal conduction, consequently exhibiting approximate average penetration rates and a similar time profile of changes in the pressure recorded by the force sensing device. Different shorter times, even by several hundred %, and also different changes in time of the pressure force exerted by the sample penetrating element against the force sensing device are exhibited by the doped waxes.

Signals from the optocouplers determine the moment when the sample penetrating element reaches the starting and final positions. Such time measurements transmitted to the microcontroller are differentiated and compared to the penetration time range adopted for the standard e.g. the natural wax. The pressure of the sample container against the force sensing device, which varies during the measurement, is recorded at even time intervals and converted into a final value, which comprises characteristics of the given wax enabling determination of a percent deviation of the result from a result expected for the standard wax. The result of the measurement is displayed on an LCD screen of the display as an average percent value representing relative deviation from the analogous time for the standard wax, as well as by one of three colors of the electroluminescent LED RGB diode. Each of the colors is assigned to a range of numeric values displayed on the screen, which means a wax close to the standard or illustrates presence of modifying substances.

The invention is disclosed in the following embodiment and on the drawing, wherein fig. 1 illustrates the device in a cross-sectional view.

The device for analysis of waxes according to the invention is constructed of a base, with a display fixed on its front panel as an LCD screen and a LED RGB diode and an internal chamber, where the power unit with the microcontroller is provided. To control the mechanical part of the device, the universal, properly programmed Arduino Mega 2560 microcontroller was used, and controlled, stabilized DC power units were used for supplying power to the heater and the stepper motor. At the surface of the base 1 a holder is provided with a support 2 for a container 3 with a tested wax sample having horizontal incisions 4 located symmetrically on opposite faces of the side surface, under which incisions shields 5 are arranged to protect the lower part of the holder against contamination with the wax. Above the holder, vertically in the middle, a mechanism is installed in a form of a tractive screw 6 connected with a stepper motor, and terminated at the bottom with the sample-penetrating element 7 in a form of a copper knife, with the heating coil 8 with a temperature sensor 9 being provided above it. Precise sliding movement of a knife penetrating the sample is controlled by the motor 10 and stabilized by two arms of a guide 11, with loosely set tips moving along vertical rods 12 attached to the base. At the bottom of the holder, on a gasket 13, a force sensing device 14 connected to the microcontroller is installed. All pieces of the holder are shielded with a panel 15 fixed to the base with a temperature sensor 16 also connected to the microcontroller. A post with two optocouplers 17 is also mounted on the base, the whole being enclosed in a casing with an opening window at the front panel to provide access to the holder designed for inserting the sample. Optionally, the casing is provided with open-work walls with a fan 18 and a Peltier module with a heater 19 controlled by the microcontroller.

## Claims

1. A device for analysis of waxes constructed of a base, with a display fixed on its front panel and an internal chamber, where a power unit with a microcontroller is provided to control operation of the device, wherein at the upper surface of the base (1) a holder is provided with a support (2) for a container (3) with a tested wax sample, with vertical incisions (4) located symmetrically on opposite faces of the side surface, under which incisions shields (5) are arranged, and above it vertically in the middle, a mechanism is installed in a form of a tractive screw (6) connected with a stepper motor, and terminated at the bottom with the sample-penetrating element (7), with a heating coil (8) with a temperature sensor (9) being provided above it, which sliding movement is controlled by the motor (10) and stabilized by two arms of a guide (11), with tips moving along vertical rods (12) attached to the base, and at the bottom of the holder, on a gasket (13), a force sensing device (14) is installed which is connected to the microcontroller, and elements of the holder are shielded with a panel (15) fixed to the base, with a temperature sensor (16) also connected to the microcontroller, and in addition on the base a post is mounted with two optocouplers (17), with the whole being enclosed in a casing with an opening window at the front panel to provide access to the holder designed for inserting the sample.

2. The device according to claim 1 **characterized in that** the element (7) penetrating the sample is in a form of a heated rod, tube or plane e.g. a knife, or a vertical needle or a rod with a sharpened tip and made of a material with good heat conduction.

3. The device according to claim 1 **characterized in that** the casing is provided with open-work walls with a fan (18) and a Peltier module with a heater (19), controlled by the microcontroller.

## Patentansprüche

1. Gerät für Wachsanalyse, aufgebaut aus einer Basis mit einer an ihrer Frontplatte befestigten Anzeige und einer inneren Kammer, in der eine Stromversorgungseinheit mit einem Mikrocontroller zur Steuerung des Betriebs des Geräts vorgesehen ist, wobei an der oberen Oberfläche der Basis (1) ein Halter mit einer Stütze (2) für einen Behälter (3) mit einer zu prüfenden Wachsprobe und mit symmetrisch auf gegenüberliegenden Seitenflächen angeordneten vertikalen Einschnitten (4), vorgesehen ist, und unter der Einschnitten Schilde (5) angeordnet sind, und darüber vertikal in der Mitte ein Mechanismus in einer Form von einer mit einem Schrittmotor verbundenen Zugschraube (6), die unten mit dem Probendurchdringungselement (7) endet, installiert ist, wobei darüber eine Heizspule (8) mit einem Temperatursensor (9) vorgesehen ist, deren Gleitbewegung durch den Motor (10) gesteuert und durch zwei Arme einer Führung (11) stabilisiert wird, wobei die Spitzen sich entlang vertikaler Stangen (12) bewegen, die an der Basis befestigt sind, und an der Unterseite des Halters auf einer Dichtung (13) ein Kraftsensor (14) installiert ist, der mit dem Mikrocontroller verbunden ist, und die Elemente des Halters mit einer an der Basis befestigten Platte (15) abgeschirmt sind, wobei auch ein Temperatursensor (16) zum Mikrocontroller verbunden ist, und übrigens auf der Basis ein Pfosten mit zwei Optokopplern (17) montiert ist, und das Ganze in einem Gehäuse mit einem zu öffnenden Fenster an der Frontplatte eingeschlossen ist, um ein Zugang zum Halter, der zum Einsetzen der Probe vorgesehen ist, zu ermöglichen.

2. Gerät nach Anspruch 1, **dadurch gekennzeichnet, dass** das die Probe durchdringende Element (7), die Form eines beheizten Stabs, Rohrs oder einer beheizten Ebene, z.B. eines Messers, einer senkrechten Nadel oder eines Stabs mit scharfer Spitze hat, und aus einem gut wärmeleitenden Material hergestellt ist.

3. Gerät nach Anspruch 1, **dadurch gekennzeichnet, dass** das Gehäuse mit durchbrochenen Wänden mit einem Ventilator (18) und einem Peltier-Modul mit einer durch einen Mikrocontroller gesteuerten Heizung (19) versehen ist.

## Revendications

1. Dispositif d'analyse de cire construit à partir d'une base, avec un afficheur fixé sur son panneau avant et une chambre interne, où une unité d'alimentation avec un microcontrôleur est pourvue pour contrôler le fonctionnement du dispositif, dans lequel à la surface supérieure de la base (1) une poignée est pourvue avec un support (2) pour un récipient (3) contenant l'échantillon de cire testée, avec des incisions verticales (4) située symétriquement sur les faces opposées de la surface latérale, sous lesquelles capots d'incisions (5) sont disposés, et au-dessus de la base, verticalement au milieu, un mécanisme en forme de vis de traction (6) relié à un moteur pas à pas, est pourvu, qui termine en bas par un élément pénétrant l'échantillon (7), avec un chauffage bobine (8) surmontée d'un capteur de température (9), dont le mouvement est contrôlé par le moteur (10) et stabilisé par deux bras d'un guide (11), dont les pointes se déplacent le long de tiges verticales (12) fixées sur la base, et au bas de la poignée, sur un joint (13), un dispositif de détection de force (14) est installé qui est connecté au microcontrôleur, et les éléments de la poignée sont protégés par un panneau (15) fixé au base, avec un capteur de température (16) également connecté au microcontrôleur, et en plus sur la base un poteau avec deux optocoupleurs (17) est monté, le tout étant enfermé dans un boîtier avec une fenêtre ouvrant sur le panneau avant pour fournir accès à la poignée prévu pour l'insertion de l'échantillon.

2. Dispositif selon la revendication 1, **caractérisé en ce que** l'élément (7) qui pénètre dans l'échantillon est sous la forme d'une tige, d'un tube ou d'un plan chauffé, par exemple un couteau, ou une aiguille verticale ou une tige avec une pointe aiguisée, et est fait en matériau à bonne conductivité thermique.

3. Dispositif selon la revendication 1, **caractérisé en ce que** le boîtier est muni de parois ajourées avec un ventilateur (18) et un module Peltier avec un réchauffeur (19), contrôlé par le microcontrôleur.
